# EUROPEAN PATENT APPLICATION

(11) **EP 1 938 749 A2**
(43) Date of publication of application: **02.07.2008**
(21) Application number: 07255014.8
(22) Date of filing: 21.12.2007
(51) Int. Cl.: A61B 5/11

(54) **Apparatus for monitoring the range of motion of a joint**

(30) Priority: 27.12.2006 US 616456
(71) Applicant: DePuy Products, Inc., Warsaw, IN 46581 (US)
(72) Inventor: Woods, Sherrod A., Warsaw IN 46582 (US); Sullivan, Thomas J., Princeton, NJ 08540 (US); Hastings, Robert S., Warsaw IN 46582 (US); Sherman, Jason T., Warsaw IN 46580 (US); Caylor, Edward J III, FT Wayne IN 46814 (US)
(74) Representative: Belcher, Simon James

(57) **Abstract**

A system for monitoring the range of motion of a patient's knee includes a knee sleeve, a number of sensor circuits coupled to the knee sleeve, and a communication circuit coupled to the knee sleeve and electrically coupled to the sensor circuits. Each sensor circuit includes a sensor configured to generate data indicative of the position of the respective sensor. The communication circuit is configured to wirelessly transmit the data to a computer.

## Description

The present invention relates to apparatus and systems for determining the range of motion of a joint of a patient.

Physical therapy is one treatment option used to treat injuries and disorders of a patient's joint, such as the patient's knee. Physical therapy typically includes a number of exercises that must be performed by the patient. For an increased rate of rehabilitation, the patient may be instructed by a healthcare provider (for example, an orthopaedic surgeon, physical therapist, etc) to perform such exercises in a regimented manner over a predetermined period of time. If the patient fails to perform the assigned exercises or performs the exercises incorrectly, rehabilitation of the patient's joint may be delayed.

Typically, the patient is required to travel to the healthcare provider's office to perform the exercises under the supervision of the healthcare provider. Alternatively, when the patient is immobile, the healthcare provider may travel to the residence of the patient to monitor the performance of the assigned exercises by the patient. In this way, the healthcare provider may verify that the patient is performing the exercises in a routine and correct manner.

In one aspect, the invention provides apparatus for monitoring the range of motion of a patient's joint includes a sleeve configured to be worn around the joint. The sleeve includes a superior half and an inferior half defined by a bisecting plane. The apparatus may also include a first sensor circuit coupled to the superior half of the sleeve. The first sensor circuit may include a first sensor configured to generate first data indicative of the position of the first sensor. The apparatus may also include a second sensor circuit coupled to the inferior half of the sleeve. The second sensor circuit may include a second sensor configured to generate second data indicative of the position of the second sensor. Additionally, the apparatus may include a communication circuit coupled to the sleeve and electrically coupled to the first sensor circuit and the second sensor circuit. The communication circuit may be configured to transmit the first and second data.

The first sensor circuit, the second sensor circuit, and/or the communication circuit may be removably coupled to the sleeve. The first sensor circuit and/or the second sensor circuit may include a third sensor configured to generate data indicative of the temperature (e.g., skin temperature) of the patient. Additionally, the first sensor circuit and/or the second sensor circuit may include a fourth sensor configured to generate data indicative of the heart rate of the patient. In such embodiments, the communication circuit may be configured to transmit the temperature data and/or the heart rate data. The communication circuit may include a memory device in some embodiments. In such embodiments, the communication circuit may be configured to store the first data, second data, the temperature data, and/or the heart rate data in the memory device. The communication circuit may also be configured to retrieve stored position data, temperature data, and/or heart rate data from the memory device and transmit such data.

In some embodiments, the sleeve may include an aperture. In such embodiments, the first sensor circuit and/or the second sensor circuit may be sized to be positioned in the aperture such that a portion of the sensor circuit is in contact with the skin of the patient when the sleeve is worn by the patient. For example, in some embodiments, the superior half of the sleeve may include a first aperture and the inferior half of the sleeve may include a second aperture. The first sensor circuit may be sized to be positioned in the first aperture such that a portion of the first sensor circuit is in contact with the skin of the patient when the sleeve is worn by the patient and the second sensor circuit is sized to be positioned in the second aperture such that a portion of the second sensor circuit is in contact with the skin of the patient when the sleeve is worn by the patient.

The apparatus may include a third sensor circuit and a fourth sensor circuit coupled to the superior and inferior half of the sleeve, respectively. The third sensor circuit may include a third sensor configured to generate third data indicative of the position of the third sensor. The fourth sensor circuit may include a fourth sensor configured to generate fourth position data indicative of the position of the fourth sensor. In such embodiments, the first sensor circuit and the second sensor circuit may be coupled to a lateral side of the sleeve and the third sensor circuit and the fourth sensor circuit may be coupled to an anterior side of the sleeve.

In another aspect, the invention provides a system for monitoring the range of motion of a patient's joint, which can include a sleeve, a first sensor coupled to the sleeve, a second sensor coupled to the sleeve, and a communication circuit coupled to the sleeve and electrically coupled to the first sensor and the second sensor. The first sensor may be configured to generate first data indicative of the position of the first sensor. The second position sensor may be configured to generate second data indicative of the position of the second sensor. The communication circuit may be configured to wirelessly transmit the first and second data.

The system may also include a first computer configured to determine third data indicative of the position of a femur of the patient based on the first data. The first computer may also be configured to determine fourth data indicative of the position of a tibia of the patient based on the second data. Additionally, the first computer may be configured to display indicia of the femur of the patient on a display device in a position based on the third data and/or indicia of the tibia of the patient on the display device in a position based on the fourth data. The first computer may also be configured to determine a cyclic count indicative of the number of times the patient's joint is moved between a flexion angle and an extension angle within a period of time based on the first data and the second data.

The system can include a second computer. The second computer may be configured to receive the first data and the second data from the communication circuit and transmit the first data and the position data to the first computer over a network. Additionally or alternatively, the second computer may be configured to display indicia of the femur of the patient on a display device in a position based on the third data and indicia of the tibia of the patient on the display device in a position based on the fourth data. In such embodiments, the first computer may be configured to transmit the first data and the second data to the second computer over a network. Additionally, the first and/or second computer may include a portable media device in some embodiments. In such embodiments, the second computer may be configured to receive the first data and the second data from the communication circuit and store the first data and the position data on the portable media device. Additionally, in such embodiments, the first computer may be configured to retrieve the first and second data from the portable media device and display indicia of the femur of the patient on a display device in a position based on the third data and indicia of the tibia of the patient on the display device in a position based on the fourth data.

The system can include a third sensor coupled to the sleeve and a fourth sensor coupled to the sleeve. The third position sensor may be configured to generate fifth data indicative of the position of the third sensor. The fourth position sensor may be configured to generate sixth data indicative of the position of the fourth sensor. In such embodiments, the first and third sensors may be coupled to a lateral side of the sleeve and the second and fourth sensors may be coupled to an anterior side of the sleeve. Additionally, the communication circuit may be configured to wirelessly transmit the first, second, fifth, and sixth data.

The system and apparatus of the invention can be used in a method for monitoring the range of motion of a patient's joint defined by a first bone and a second bone may include determining first data indicative of the position of a first sensor coupled to a superior half of a garment wearable by the patient, determining second data indicative of the position of a second sensor coupled to an inferior half of the sleeve, and transmitting the first and second data to a first computer. The method may also include determining third data indicative of a position of the first bone based on the first data and fourth data indicative of a position of the second bone based on the second data. The determination of the third data and fourth data may be performed by the first computer. The method may further include determining data indicative of the range of motion of the patient's joint based on the third data and the fourth data. In some embodiments, the method may also include displaying a rendered image of the first bone of the patient on a display screen in a position based on the third data and a rendered image of the second bone of the patient on the display screen in a position based on the fourth data. Additionally, the method may include storing the first and second data on a portable media device and retrieving the stored first and second data from the portable media device.

The method can include the step of transmitting the first data and the second data from the first computer to a second computer over a network. In such embodiments, the determining step and the displaying step are performed by the second computer. Yet further, the method may include determining temperature data indicative of the temperature of the patient and transmitting the temperature data to the first computer. Additionally, the method may include determining a cycle count indicative of the number of times the patient's joint is moved between a flexion position and an extension position within a period of time.

Embodiments of the invention will now be described by way of example with reference to the accompanying drawings, in which:
FIG. 1 is a lateral elevation view of one embodiment of an apparatus for determining the range of motion of a patient's knee;
FIG. 2 is an anterior elevation view of the apparatus of FIG. 1;
FIG. 3 is a cross-sectional view of one embodiment of a knee sleeve of the apparatus of FIG. 1;
FIG. 4 is a cross-sectional view of another embodiment of the knee sleeve of the apparatus of FIG. 1;
FIG. 5 is a simplified block diagram of one embodiment of the sensing circuitry of the apparatus of FIG. 1;
FIG. 6 is a simplified block diagram of one embodiment of a sensor circuit of the sensing circuitry of FIG. 3;
FIG. 7 is a simplified block diagram of a system for determining the range of motion of a patient's knee;
FIG. 8 is a simplified flow diagram of an algorithm for transmitting sensor data, which may be executed by a communication circuit of the system of FIG. 7;
FIG. 9 is a simplified flow diagram of an algorithm for receiving and transmitting sensor data, which may be executed by a patient computer of the system of FIG. 7; and
FIG. 10 is a simplified flow diagram of an algorithm for determining the range of motion of the patient's knee, which may be executed by a healthcare provider computer of the system of FIG. 7.

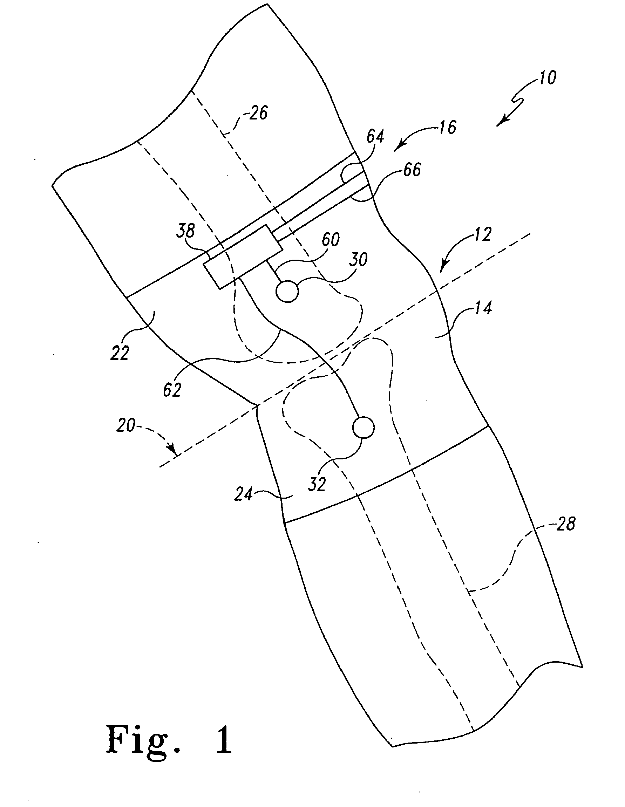

Referring to the drawings, FIG. 1 show apparatus 10 for determining the range of motion of a patient's knee 12 includes a knee sleeve 14 and sensing circuitry 16 coupled to the knee sleeve 14. The knee sleeve 14 includes a superior half 22 and an inferior half 24 defined by a bisecting plane 20. The knee sleeve 14 has a substantially cylindrical shape having an opening at each end and an inner cavity defined therebetween through which the leg of the patient is inserted when the knee sleeve 14 is worn by the patient.

As shown in FIG. 1, when the knee sleeve 14 is worn by the patient, the superior half 22 of the knee sleeve covers a portion of the distal end of the patient's femur 26 and the inferior half 24 covers a portion of the proximal end of the patient's tibia 28. The knee sleeve 14 may be formed from any flexible material that allows the patient to move the knee joint 12 between a flexion position and an extension position. For example, the knee sleeve 14 may be formed from a stretchable, form-fitting textile such as a neoprene material, nylon, a spandex material such as LycraTM, or the like. Additionally, in some embodiments, the knee sleeve 14 may be formed from a sterilized material such that the knee sleeve 14 may be worn by the patient soon after the surgical procedure and/or during post-surgery recovery.

The knee sleeve 14 may be sized based on one or more parameters such as the physical size of the patient. For example, the knee sleeve 14 is sized such that the knee sleeve 14 covers only the knee joint region of the patient's leg. That is, when the knee sleeve 14 is worn by the patient, the superior half 22 of the knee sleeve 14 does not extend up to the groin region of the patient and the inferior half 24 does not extend down to the ankle region of the patient. Because the knee sleeve 14 is worn only around the knee joint region of the patient's leg, the natural movement of the patient's joint is not substantially affected by the use of the knee sleeve 14 unlike a full-leg sleeve or full body suit, which extends from groin-to-ankle. Although the knee sleeve 14 does not substantially affect the natural movement of the patient's joint, the knee sleeve 14 may provide some amount of additional support to the knee joint in some embodiments.

In the embodiment shown in the drawings, the sensing circuitry 16 includes a number of sensor circuits 30, 32, 34, 36 and a communication circuit 38. In some embodiments, the sensor circuits 30, 32, 34, 36 and the communication circuit 38 are incorporated into the knee sleeve 14. For example, the circuits 30, 32, 34, 36, 38 may be woven into the material forming the knee sleeve 14 or otherwise attached to the knee sleeve 14 in a non-removable manner using, for example, a permanent adhesive or the like.

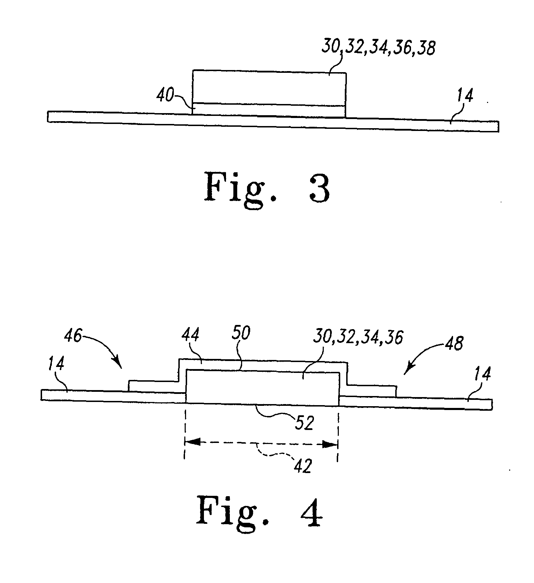

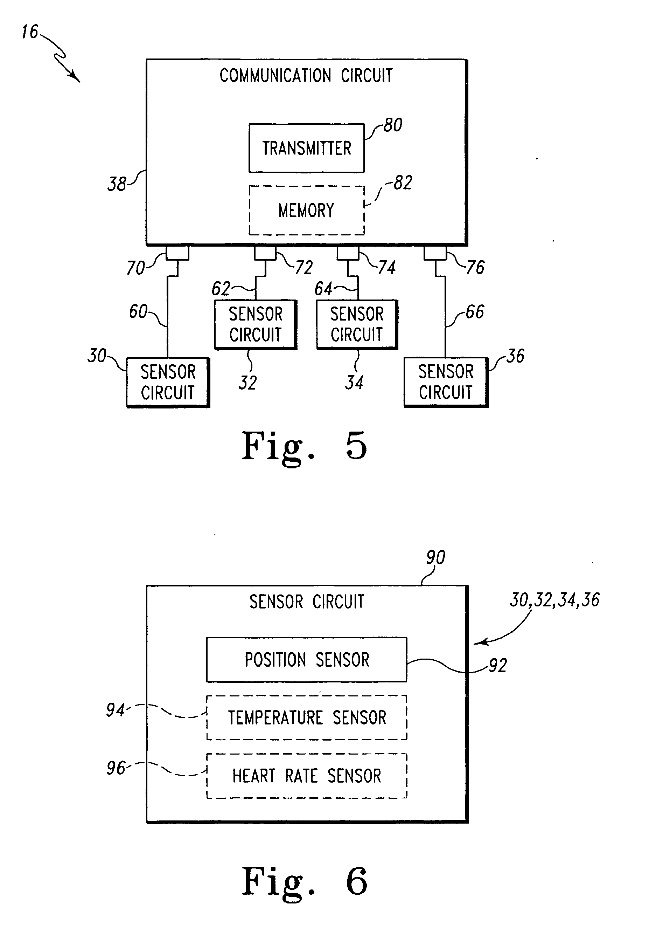

Alternatively, in other embodiments, the sensor circuits 30, 32, 34, 36 and the communication circuit 38 may be removably attached to the knee sleeve 14. For example, as shown in FIG. 3, the sensor circuits 30, 32, 34, 36 and/or the communication circuit 38 may be removably attached to the knee sleeve 14 using an attaching member 40, such as a hook-and-loop material or other non-permanent adhesive. In such embodiments, the attaching member 40, or a portion thereof, may be secured to a housing (see FIG. 6) of the circuit 30, 32, 34, 36, 38. In use, the sensor circuits 30, 32, 34, 36 and/or the communication circuit 38 may be attached to the knee sleeve 14 by pressing the attaching member 40 onto the knee sleeve 14. In such embodiments, the sensor circuits 30, 32, 34, 36 and the communication circuit 38 may be coupled to the knee sleeve 14 during use and decoupled thereform when not in use. Additionally, in embodiments in which the circuits 30, 32, 34, 36, 38 are removable from the knee sleeve 14, the circuits 30, 32, 34, 36, 38 may be reusable while the knee sleeve 14 is disposed of after each use or otherwise periodically.

In some embodiments, as shown in FIG. 4, the knee sleeve may include a number of apertures 42. The apertures 42 are sized to receive at least a portion of one of the sensor circuits 30, 32, 34, 36. That is, when the sensor circuit 30, 32, 34, 36 is coupled to the knee sleeve 14, a portion of the sensor circuit 30, 32, 34, 36 is positioned in the aperture 42. The sensor circuit 30, 32, 34, 36 is held in place via an attaching member 44, which wraps over the sensor circuit 30, 32, 34, 36 and contacts a portion of the knee sleeve 14 at distal ends 46, 48. The attaching member 44 may be similar to the attaching member 40 described above with reference to FIG. 3. For example, the attaching member 44 may be secured to a top surface 50 of the sensor circuit 30, 32, 34, 36 and formed from a hook-and-loop material.

When the sensor circuit 30, 32, 34, 36 is positioned in the aperture 42, a bottom surface 52 of the sensor circuit 30, 32, 34, 36 may be positioned in contact with the skin of the patient. Such positioning of the sensor circuit 30, 32, 34, 36 allows measurements of particular parameters of the patient, such as the patient's temperature and/or heart rate, in some embodiments as discussed in more detail below with reference to FIG. 6. The knee sleeve 14 may include any number of apertures 42. In one particular embodiment, the knee sleeve 14 includes one aperture 42 for each sensor circuit 30, 32, 34, 36. Additionally, the apertures 42 may be defined in knee sleeve 14 in predetermined locations such that the sensor circuits 30, 32, 34, 36 are similarly located when coupled thereto. For example, as discussed in more detail below, one or more apertures 42 may be defined on a lateral side of the knee sleeve 14 and one or more additional apertures 42 may be defined on the anterior side of the knee sleeve 14.

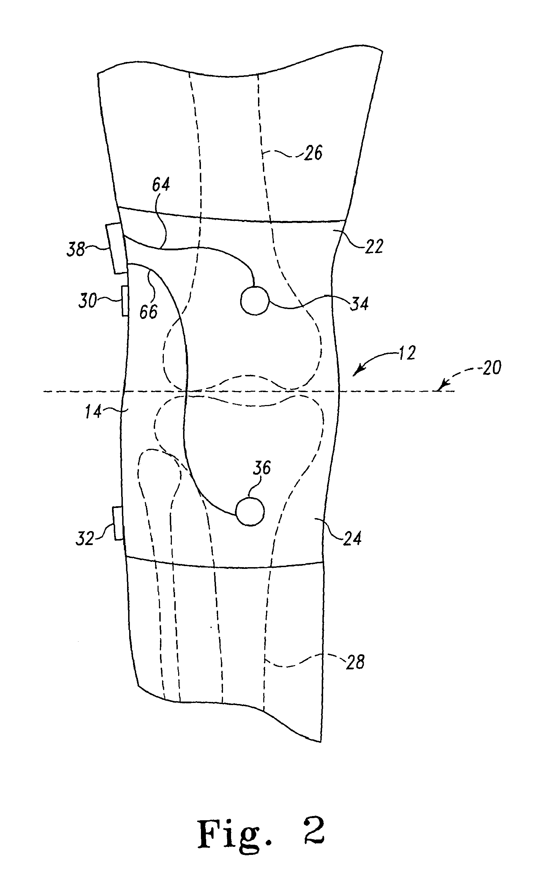

Referring back to FIGS. 1 and 2, the sensor circuits 30, 32, 34, 36 are positioned on the knee sleeve 14 such that one or more of the sensor circuits 30, 32, 34, 36 is located on each side (i.e., the superior and inferior sides) of the knee joint 12 of the patient. In particular, sensor circuit 30 is coupled on the lateral side of the superior half 22 of the knee sleeve 12 and the sensor circuit 32 is coupled on the lateral side of the inferior half 24 of the knee sleeve as shown in FIG. 1. Additionally, as shown in FIG. 2, the sensor circuit 34 is coupled on the anterior side of the superior half 22 of the knee sleeve 12 and the sensor circuit 36 is coupled on the anterior side of the inferior half 24 of the knee sleeve 12. Although the illustrative apparatus 10 includes four sensor circuits, it should be appreciated that in other embodiments a greater or lesser number of sensor circuits may be used. For example, in some embodiments, only two sensor circuits are used and each sensor circuit is positioned on one side of the knee joint 12 (e.g., similar to sensor circuits 30, 32). However, by using additional sensor circuits, such as sensor circuits 34, 36, an amount of redundancy and an improvement in measurement accuracy may be achieved with the apparatus 10.

In the embodiment shown in the drawings, the sensor circuits 30, 34 and the sensor circuits 32, 36 are positioned on the lateral and anterior side of the knee sleeve 14 to reduce the likelihood that any one of the sensor circuits 30, 32, 34, 36 obstructs the normal movement of the patient or becomes dislodged from the knee sleeve 14. For example, such positioning of the sensor circuits 30, 32, 34, 36 may reduce the likelihood that the sensor circuits 30, 32, 34, 36 inadvertently become dislodged from the knee sleeve 14 by movement of the patient from, for example, being repeatedly hit or rubbed by the leg of the patient as may be the case if sensor circuits were located on the medial side of the knee sleeve 14. However, in other embodiments, the sensor circuits 30, 34 and 32, 36 may be positioned in other locations on the knee sleeve 14.

Each of the sensor circuits 30, 32, 34, 36 is electrically coupled to the communication circuit 38 via a number of communication links 60, 62, 64, 66, respectively. The communication links 60, 62, 64, 66 may be embodied as any type of communication link capable of providing electrical communication between the sensor circuits 30, 32, 34, 36 and the communication circuit 38. For example, the communication links 60, 62, 64, 66 may be embodied as any number of wires, cables, fiber optic cables, and/or the like. In some embodiments, the sensor circuits 30, 32, 34, 36 may be removably coupled to the communication circuit 38. For example, as shown in FIG. 5, the sensor circuits 30, 32, 34, 36 may be communicatively coupled to the communication circuit 38 via connectors 70, 72, 74, 76. In this way, individual sensor circuits that become damaged over time may be replaced without the requirement of replacing the communication circuit 38. In addition, only those sensor circuits required for the particular application or implementation may be coupled to the communication circuit 38. For example, in those embodiments including only two sensor circuits rather than four, the sensor circuits 30, 32 may be coupled to the communication circuit 38 and to the knee sleeve 14, while the sensor circuits 34 and 36 are decoupled from the communication circuit 38 and removed.

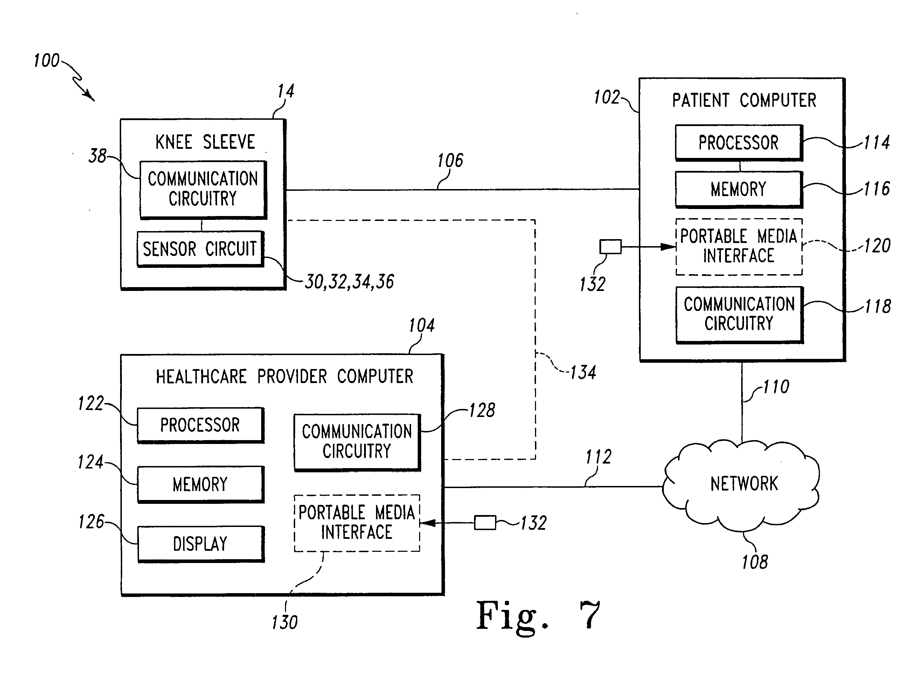

As shown in FIG. 5, the communication circuit 38 includes a transmitter 80. The transmitter 80 is configured to receive the sensor data signals from the sensor circuits 30, 32, 34, 36 and transmit the sensor data signals to a receiver such as a remote computer as discussed below in more detail with reference to FIG. 7. The transmitter 80 may be embodied as any transmitter circuitry capable of wirelessly transmitting the data signals received from the sensor circuits 30, 32, 34, 36. The transmitter 80 is sized to allow the communication circuit 38 to be coupled to or incorporated in the knee sleeve 14 while not falling off due to gravity or use or otherwise causing the knee sleeve 14 to become improperly positioned during use. In some embodiments, the communication circuit 38 also includes a memory device 82. The memory device 82 may be embodied as any type of memory device such as, for example, a random access memory (RAM) device. In such embodiments, the transmitter 80 or associated circuitry may be configured to store the sensor data received from the sensor circuits 30, 32, 34, 36 in the memory device 82. In addition, the transmitter 80 or associated circuitry may be configured to retrieve stored sensor data from the memory device 82 and transmit the stored sensor data to a receiver such as a remote computer.

Although the communication circuit 38 is shown in FIG. 5 as a single circuit, in some embodiments, the communication circuit 38 may be embodied as any number of transmitters similar to transmitter 80, which may or may not be communicatively coupled to each. In such embodiments, each transmitter may be electrically coupled to a respective one of the sensor circuits 30, 32, 34, 36 and may further form a portion of the respective sensor circuit 30, 32, 34, 36 in some embodiments. For example, in one particular embodiment, the communication circuit 38 is formed from a first transmitter electrically coupled to the sensor circuit 30, a second transmitter electrically coupled to the sensor circuit 32, a third transmitter electrically coupled to the sensor circuit 34, and a fourth transmitter electrically coupled to the sensor circuit 36. Additionally, in such embodiments, any one or more of the sensor circuits 30, 32, 34,36 may include a memory device similar to memory device 82.

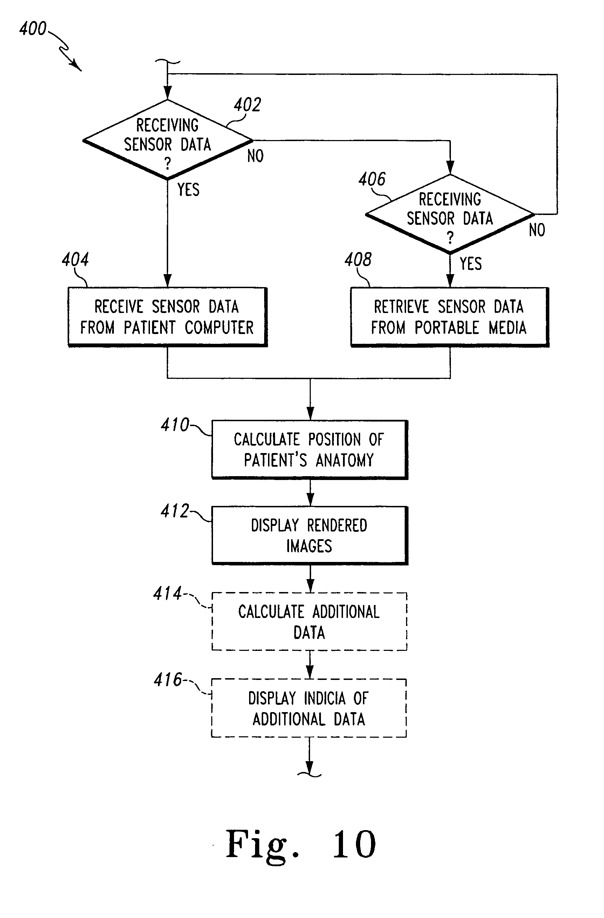

Referring now to FIG. 6, each of the sensor circuits 30, 32, 34, 36 includes a housing 90 and a position sensor 92. The housing 90 may be formed from any material capable of supporting the position sensor 92 and associated circuitry and being coupled to the knee sleeve 14. In one particular embodiment, the housing 90 is formed from a plastic material, but other materials may be used in other embodiments. In embodiments in which the knee sleeve 14 includes apertures 42, the housing 90 or a portion thereof is sized to be positioned in one of the apertures 42. The position sensor 92 may be embodied as any sensor capable of generating sensor data indicative of the position of the sensor 92. As used herein, the term "position" is intended to mean the spatial location of an object (for example, the sensor 92) relative to a reference point and/or the spatial orientation of an object (for example, the sensor 92) relative to a reference axis. For example, in one particular embodiment, the position sensor 92 may be embodied as a microelectro-mechanical system (MEMS) sensor, such as an accelerometer, configured to generate sensor data indicative of the orientation of the position sensor 92 with respect to the Earth's gravitational field. However, in other embodiments, the position sensor 92 may be embodied as any other type of position sensor, such as optical and/or magnetic position sensors, configured to generate data indicative of the location and/or the orientation of the sensor from which the position of an associated bone of the patient may be determined as described in more detail below with reference to FIG. 10.

In some embodiments, the sensor circuits 30, 32, 34, 36 may also include a temperature sensor 94 and/or a heart rate sensor 96. The temperature sensor 94 may be embodied as any type of temperature sensor capable of generating sensor data indicative of the temperature of the patient. Similarly, the heart rate sensor 96 may be embodied as any type of heart rate sensor capable of generating a sensor data indicative of the heart rate of the patient. In such embodiments, the temperature sensor 94 and/or the heart rate sensor 96 may be positioned in the housing 90 such that the temperature sensor 94 and heart rate sensor 96 (or portions thereof) are in contact or positioned closely to the skin of the patient. For example, in embodiments in which the knee sleeve 14 includes the apertures 42, the temperature sensor 94 and/or the heart rate sensor 96 may be positioned in the housing 90 such that a portion of the sensors 94, 96 are in contact with or otherwise close to the skin of the patient. Additionally, in embodiments in which the sensor circuits 30, 32, 34, 36 include a temperature sensor 94 and/or a heart rate sensor 96, such sensors 94, 96 may be positioned on a circuit board (not shown) or other substrate in common with the position sensors 92. Additionally, the sensor circuits 30, 32, 34, 36 may include other circuitry not shown in FIG. 6. For example, the sensor circuits 30, 32, 34, 36 may include transmitter circuitry, amplification circuitry, and/or the like for propagating the sensor data to the communication circuit 38 via the communication links 60, 62, 64, 66.

Referring now to FIG. 7, a system 100 for monitoring the range of motion of a patient's joint includes the knee sleeve 14, a patient computer 102, and a healthcare provider computer 104. The patient computer 102 is communicatively coupled to the knee sleeve 14 via a number of communication links 106. The communication links 106 may be embodied as any type of communication links capable of facilitating communication between the sensing circuit 16 of the knee sleeve 14 and the patient computer 102. For example, the communication links 106 may be embodied as any number of cables, wires, fiber optic cables, wireless signals, and/or the like. The patient computer 102 is also communicatively coupled to the healthcare provider computer 104 via a network 108. The network 108 may be embodied as any type of communication network capable of facilitating communication between the patient computer 102 and the healthcare provider computer 104. For example, the network 108 may be embodied as a wide area network (WAN), a local area network (LAN), or form a portion of a publicly-accessible, global network such as, for example, the Internet. In addition, the network 108 may be a wired network, a wireless network, or a combination thereof. As such, the network 108 may include any number of devices for providing communication between the computers 102, 104 such as routers, switches, computers, communication links, and the like.

The patient computer 102 is coupled to the network 108 via a number of communication links 110. Similarly, the healthcare provider computer 104 is coupled to the network 108 via a number of communication links 112. The communication links 110, 112 may be embodied as any type of communication links capable of providing communication between the patient computer 102 and the healthcare provider computer 104. For example, the communication links 110, 112 may be embodied as any number of cables, wires, fiber optic cables, wireless signals, and/or the like.

The patient computer 102 includes a processor 114 and a memory device 116. The processor 115 may be embodied as any type of processor including, for example, discrete processing circuitry (for example, a collection of logic devices), general purpose integrated circuit(s), and/or application specific integrated circuit(s) (ASICs). The memory device 116 may be embodied as any type of memory device and may include one or more memory types, such as, random access memory (RAM) and/or read-only memory (ROM). In addition, the patient computer 102 may include other devices and circuitry typically found in a computer for performing the functions described herein such as, for example, a hard drive, input/output circuitry, and the like. As such, the patient computer 102 may be embodied as any type of computer or computing device capable of receiving data from the knee sleeve 14 and transmitting the data to the healthcare provider computer 104. For example, the computer 102 may be embodied as a typical desktop, laptop computer, or personal digital assistant (PDA) device equipped with a display screen, keyboard, and other devices and circuitry typically found in a desktop computer, laptop computer, or PDA. Alternatively, the patient computer 102 may be embodied as an application specific computer or computer device configured to perform the functions described herein.

The patient computer 102 also includes communication circuitry 118 to facilitate communication with the sensing circuit 16 of the knee sleeve 14 and the healthcare provider computer 104. As such, the communication circuitry 118 may include transmitter and/or receiver circuitry. Additionally, the communication circuitry may be configured to communicate with the sensing circuit 16 of the knee sleeve 14 and/or the healthcare provider computer 104 using wired or wireless communication protocols. As such, the communication circuitry 118 may be embodied as wired and/or wireless communication circuitry. In one particular embodiment, the communication circuitry 118 is configured to wirelessly receive sensor data from the communication circuitry 38 of the knee sleeve 14 via the communication links 106 and transmit the sensor data and/or other data to the healthcare provider computer 104 via the communication links 110, 112 and the network 108.

In some embodiments, the patient computer 102 may also include a portable media interface 120. The portable media interface 120 is configured to receive a portable media device 132. In the illustrative embodiment, the portable media interface 120 is embodied as a Universal Serial Bus (USB) port. However, in other embodiments, the portable media interface 120 may be embodied as any type of serial port, parallel port, flash drive port, or other data port capable of communicating with and storing data on the portable media device 132. The portable media device 132 may be embodied as any portable memory device configured for the purpose of transporting data from one computer system to another computer system. In some embodiments, the portable media memory device 132 is embodied as a removable solid-state memory device such as a removable flash memory device. For example, the portable media device 132 may be embodied as a MemoryStick™ flash memory device, a SmartMedia™ flash memory device, or a CompactFlash™ flash memory device. Alternatively, in other embodiments, the portable media device 132 may be embodied as a memory device having a microdrive for data storage. Regardless, the portable media memory device 132 is capable of storing data such as sensor data for later retrieval.

The healthcare provider computer 104 includes a processor 122, memory device 124, and a display 126. The processor 122 may be embodied as any type of processor including, for example, discrete processing circuitry (for example, a collection of logic devices), general purpose integrated circuit(s), and/or application specific integrated circuit(s) (ASICs). The memory device 124 may be embodied as any type of memory device and may include one or more memory types, such as, random access memory (RAM) and/or read-only memory (ROM). The display 126 may be embodied as any type of display or display device capable of displaying data and images to a user (for example, an orthopaedic surgeon or physical therapist) of the healthcare provider computer 104. In some embodiments, the display 126 forms an integral portion of the healthcare provider computer 104. However, in other embodiments, the display 126 may be separate from the healthcare provider computer, but communicatively coupled to it.

The healthcare provider computer 104 also includes communication circuitry 128 to facilitate communication with the patient computer 102. As such, the communication circuitry 128 may include transmitter and/or receiver circuitry. Additionally, the communication circuitry 128 may be configured to communicate with the patient computer 102 using wired or wireless communication protocols depending upon, for example, the type of network 108. For example, in embodiments in which the network 108 is a wireless network, the communication circuitry 128 may be embodied as a wireless communication circuitry.

In addition to communicating with the patient computer 102, the healthcare provider computer 104 may be configured to communicate directly with the sensing circuit 16 of the knee sleeve 14 in some embodiments. For example, when the patient is at the healthcare provider's office, the healthcare provider computer 104 may receive sensor data from the communication circuitry 38 of the knee sleeve 14 rather than from the patient computer 102, which may be remotely located from the healthcare provider's office. In such embodiments, the communication circuitry 128 includes suitable circuitry to facilitate communication with the communication circuitry 38 of the knee sleeve 14. For example, the communication circuitry 128 may include wireless communication circuitry configured to wirelessly receive sensor data from the communication circuitry 38 of the knee sleeve 14 via a wireless communication link 134.

Additionally, in some embodiments, the healthcare provider computer 104 may also include a portable media interface 130. The portable media interface 130 is configured to receive the portable media device 132. In the illustrative embodiment, the portable media interface 130 is embodied as a Universal Serial Bus (USB) port. However, in other embodiments, the portable media interface 130 may be embodied as any type of serial port, parallel port, or other data port capable of communicating with and retrieving data from the portable media device 132. As discussed above, the portable media device 132 may be embodied as any portable memory device configured for the purpose of transporting data from one computer system to another computer system.

In addition, the healthcare provider computer 104 may include other devices and circuitry typically found in a computer for performing the functions described herein such as, for example, a hard drive, input/output circuitry, and the like. As such, the healthcare provider computer 104 may be embodied as any type of computer or computing device capable of receiving data from the patient computer 102 and displaying data to a user (for example, an orthopaedic surgeon or a physical therapist). For example, the healthcare provider computer 104 may be embodied as a typical desktop or laptop computer equipped with a display screen, keyboard, and other devices and circuitry typically found in a desktop and/or laptop computer. Alternatively, the healthcare provider computer 104 may be embodied as an application specific computer or computer device configured to perform the functions described herein. Further, in some embodiments, the healthcare provider computer 104 may form a portion of a hospital network or otherwise be communicatively coupled to such a network.

In use, the patient computer 102 may be located at the residence of the patient while the healthcare provider computer 104 is located at the healthcare provider's office. Alternatively, as discussed above, the healthcare provider computer 104 may be located in a hospital or otherwise form a portion of a hospital network. In such embodiments, the healthcare provider computer 104 may be remotely accessible by the healthcare provider such as from a remote computer located in the office of the healthcare provider. The patient computer 102 is configured to receive sensor data from the sensing circuit 16 of the knee sleeve 14 via the communication link 106. The sensor data includes the data received from the respective position sensors 92 and, in some embodiments, from the temperature sensor(s) 94 and the heart rate sensor(s) 96. The patient computer 102 may be configured to store the received sensor data in the memory device 116 and/or the portable media device 120. Additionally, the patient computer 102 may be configured to transmit the sensor data to the healthcare provider computer 104 via the network 108. The patient computer 102 may transmit the data continually or periodically. That is, in some embodiments, the patient computer 102 may be configured to store the received sensor data over time and only transmit the sensor data during a predetermined time period or in response to a request received from the healthcare provider computer 104.

Regardless, once the healthcare provider computer 104 receives the sensor data, the computer 104 is configured to determine the range of motion of the patient's joint (for example, the patient knee) based on the sensor data generated by the position sensors 92. Additionally, the healthcare provider computer 104 may be configured to determine the temperature of the patient based on the sensor data generated by the temperature sensor(s) 94 and the heart rate of the patient based on the sensor data generated by the heart rate sensor(s) 96. The healthcare provider computer 104 may subsequently display an image indicative of the range of motion of the patient's joint, the patient's temperature, and/or the heart rate of the patient on the display device 126 for review by the healthcare provider (for example, orthopaedic surgeon or physical therapist). The healthcare provider computer 104 may also be configured to determine other data based on the sensor data received from the patient computer 102. For example, in some embodiments, the healthcare provider computer 104 may be configured to determine a count of the number of times that the patient's joint is cycled (for example, moved from an extension position to a flexion position and back to the extension position). In this way, the system 100 may be used by a healthcare provider to remotely monitor the range of motion of a joint of the patient along with other data related to the patient. Based on the determined range of motion data, the healthcare provider may be able to determine whether the patient is routinely performing any assigned exercises and whether the patient is performing the exercises correctly (for example, extending the joint to a full extension position).

In some embodiments, the patient computer 102 may also be configured to determine the range of motion of the patient's joint based on the sensor data generated by the position sensors 92, the temperature of the patient based on the sensor data generated by the temperature sensor(s) 94, and/or the heart rate of the patient based on the sensor data generated by the heart rate sensor(s) 96. In such embodiments, the patient computer 102 may display an image indicative of the range of motion of the patient's joint, the patient's temperature, and/or the heart rate of the patient on a display device (not shown) of the patient computer 102. In this way, the patient may monitor their physical therapy progress as indicated by such data as the range of motion of the relevant joint.

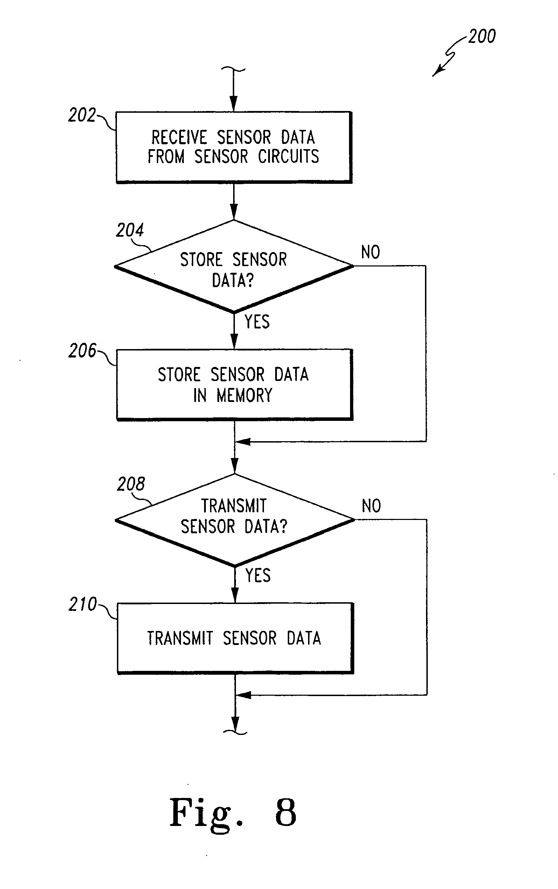

Referring now to FIG. 8, an algorithm 200 for transmitting sensor data that may be executed by the sensing circuitry 16 of the knee sleeve 14 begins with a process step 202. In process step 202, the communication circuitry 38 of the sensing circuit 16 receives sensor data from each of the sensor circuits 30, 32, 34, 36. As discussed above, the sensor data may include data from the position sensors 92, the temperature sensor(s) 94, the heart rate sensor(s) 96, and/or any other sensors included in the sensor circuits 30, 32, 34, 36. The communication circuitry 38 may continually, periodically, or selectively receive the sensor data from the sensor circuits 30, 32, 34, 36. For example, the communication circuitry 38 or associated circuitry may be configured to poll the sensor circuits 30, 32, 34, 36 periodically to receive the sensor data. Alternatively, the communication circuit may transmit a signal to each of the sensor circuits 30, 32, 34, 36 to request that the individual sensor circuit 30, 32, 34, 36 transmit the sensor data. Regardless, the communication circuit 38 receives the sensor data in process step 202.

In process step 204, the communication circuit 38 determines whether the sensor data should be stored. If so, the sensor data received from the sensor circuits 30, 32, 34, 36 is stored in the memory device 82 in process step 206. Subsequently or if the sensor data is to be stored, the algorithm 200 advances to process step 208. In process step 208, the communication circuit 38 determines whether the sensor data should be transmitted. The communication circuit 38 may be configured to transmit the data continually, periodically, or in response to a request signal received from the patient computer 102 (or from the healthcare provider computer 104 in some embodiments). For example, in some embodiments, the communication circuit 38 is configured to transmit the sensor data only during predetermined time periods. In other embodiments, the communication circuit 38 may be configured to transmit the sensor data only after receiving a request for the sensor data from the patient computer 102. Regardless, if the communication circuit 38 determines that the sensor data should be transmitted, the algorithm 200 advances to process step 210 in which the communication circuit 38 transmits the sensor data to the patient computer 102 via the communication link 106 (or to the healthcare provider computer 104 via the communication link 134). Additionally, in some embodiments such as in those embodiments in which the communication circuit 38 is configured to store received sensor data, the communication circuit 38 may retrieve the stored sensor data and transmit the stored sensor data in process step 210.

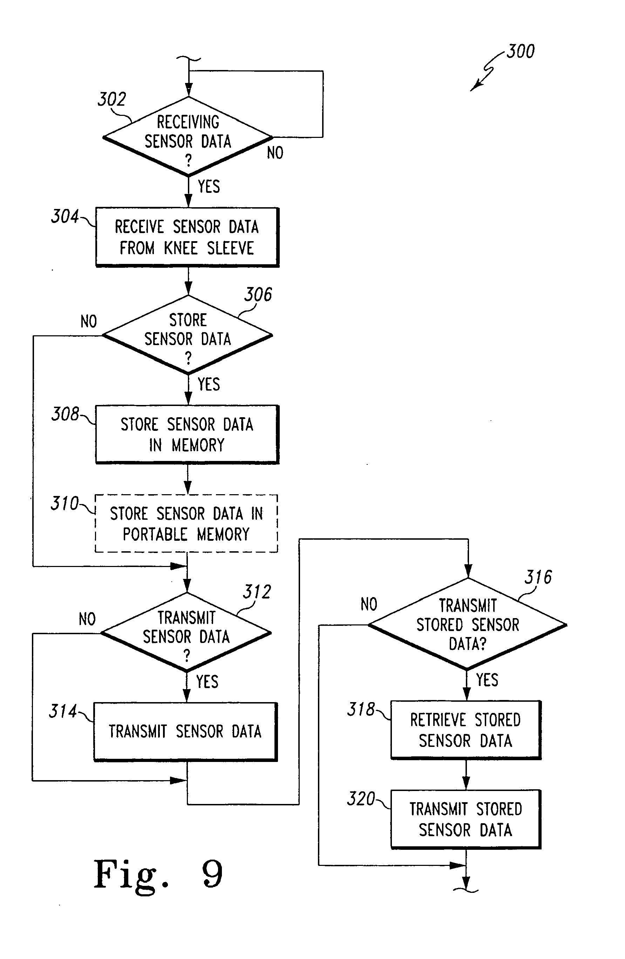

Referring now to FIG. 9, an algorithm 300 for receiving and transmitting sensor data that may be executed by the patient computer 102 begins with a process step 302. In process step 302, the patient computer 102 determines whether the communication circuitry 38 is transmitting any sensor data. As discussed above, in some embodiments, the communication circuitry 38 may be configured to transmit the sensor data continually or periodically. Alternatively, in other embodiments, the patient computer 102 is configured to transmit a request signal to the communication circuitry 38 via the communication link 106 to inform the sensing circuitry 16 that the patient computer 102 is ready to receive sensor data. Regardless, if the communication circuitry 38 is transmitting the sensor data, such sensor data is received by the patient computer 102 in process step 304. Next, in process step 306, the patient computer 102 determines whether the sensor data should be stored. The patient computer 102 may make such a determination based on predefined conditions. For example, the patient computer 102 may be programmed to store or not store the sensor data in some embodiments.

If the patient computer 102 determines that the sensor data should be stored, the patient computer 102 stores the sensor data in the memory device 116 in process step 308. Additionally, in embodiments in which the patient computer 102 includes the portable media interface 120, the patient computer 102 may be configured to store the received sensor data on the portable media device 132. Further, in some embodiments, the patient computer 102 may be configured to retrieve previously stored sensor data from the memory device 116 and store the retrieved sensor data on the portable media device 132 in process step 310.

Once the sensor data has been stored or if the patient computer 102 determines that the sensor data is not to be stored, the algorithm 300 advances to process step 312. In process step 312, the patient computer 102 determines whether the sensor data received from the communication circuit 38 of the knee sleeve 14 should be transmitted to the healthcare provider computer 104. The patient computer 102 may be configured to transmit the data continually, periodically, or in response to a request signal received from the healthcare provider computer 104. For example, in some embodiments, the patient computer 104 is configured to transmit the sensor data to the healthcare provider computer 104 only during predetermined time periods. In other embodiments, the patient computer 102 may be configured to transmit the sensor data only after receiving a request for the sensor data from the healthcare provider computer 104. Alternatively, in some embodiments, the patient computer 102 may be configured to not transmit the sensor data at all. In such embodiments, the sensor data may be transferred from the patient computer 102 to the healthcare provider computer 104 via the portable media device 132. Regardless, if the patient computer 102 determines that the sensor data should be transmitted, the algorithm 300 advances to process step 314 in which the patient computer 102 is configured to transmit the sensor data to the healthcare provider computer 104 via the communication links 110, 112 and the network 108.

Once the sensor data has been transmitted to the healthcare provider computer 104 or if the algorithm 300 determines that the sensor data should not be transmitted, the algorithm 300 advances to process step 316. In process step 316, the patient computer 102 determines whether any stored sensor data should be transmitted to the healthcare provider computer 104. If so, the algorithm 300 advances to process step 318 in which stored sensor data is retrieved from the memory device 116. As discussed above, the stored sensor data may include sensor data generated from the position sensors 92, the temperature sensor(s) 94, the heart rate sensor(s) 96, and/or any other sensors included in one or more of the sensor circuits 30, 32, 34, 36. Once the stored sensor data has been retrieved in process step 318, the stored sensor data is transmitted to the healthcare provider computer 104 via the communication links 110, 112 and the network 108 in process step 320.

In some embodiments, the patient computer 102 may be configured to retrieve the stored sensor data in response to a request signal received from the healthcare provider computer 104. In such embodiments, the patient computer 102 may retrieve the stored sensor data and transmit the stored sensor data to the healthcare provider computer 104 regardless whether the patient computer 104 is receiving sensor data from the communication circuit 38 of the knee sleeve 14. That is, although the retrieval of stored sensor data is illustrated in algorithm 300 as occurring only subsequent to the receipt of sensor data in process step 304, in other embodiments, the patient computer 104 may be configured to retrieve and transmit the sensor data at any time in response to a request signal received by the healthcare provider computer 104. Additionally or alternatively, the patient computer 102 may be configured to periodically transmit the stored data. As such, it should be appreciated that the patient computer 104 may transmit sensor data or otherwise communicate with the healthcare provider computer 104 regardless of whether any sensor data is being received from the communication circuit 38.

In some embodiments, as discussed above, the patient computer 102 may be configured to determine the range of motion of the patient's joint, the temperature of the patient, the heart rate of the patient, and/or other data based on the sensor data received in process step 304. In such embodiments, the patient computer 102 may determine the range of motion using the algorithm described in process steps 410 and 412 of algorithm 400 described below with reference to FIG. 10. Additionally, the patient computer 102 may determine other data such as the patient's temperature and/or heart rate using the algorithm described below in relation to the process steps 414 and 416 of algorithm 400. As such, it should be appreciated that in some embodiments the patient computer 102 may perform such determinations and display images indicative of such data in lieu of or in addition to the healthcare provider computer 104. Again, such a configuration allows the patient to monitor their physical therapy progress as indicated by the determined range of motion of the relevant joint.

Referring now to FIG. 10, an algorithm 400 for determining a range of motion of a patient's joint (e.g., knee joint 12) that may be executed by the healthcare provider computer 104 (and/or the patient computer 102 in some embodiments) begins with a process step 402. In process step 402, the healthcare provider computer 104 determines if sensor data is being received from the patient computer 102 via the communication links 110, 112 and the network 108 (or directly from the communication circuitry 38 via the communication link 134). As discussed above, the patient computer 102 may be configured to transmit the sensor data to the healthcare provide computer 104 continually, periodically, or in response to a request signal transmitted by the healthcare provider computer 104. Regardless, if the patient computer 102 is transmitting sensor data, such sensor data is received by the healthcare provider computer 104 in process step 404. Once the sensor data is received from the patient computer 104, the algorithm 400 advances to process step 410.

Referring back to process step 402, if sensor data is not being transmitted by the patient computer 102, the algorithm 400 advances to process step 406 in which the healthcare provider computer 104 determines whether any sensor data should be retrieved from the portable media device 132. For example, the sensor data may be stored on the portable media device 132 by the patient computer 102. Subsequently, the portable media device 132 may be brought to the healthcare providers office and inserted or otherwise communicatively coupled to the portable media interface 130 of the healthcare provide computer 104. In response, the computer 104 may be configured to prompt the healthcare provider whether any sensor data should be retrieved from the portable media device 132. If so, the algorithm 400 advances to process step 408 in which any sensor data stored on the portable media device 132 is retrieved by the healthcare provider computer 104. In this way, sensor data may be transferred from the patient computer 102 to the healthcare provider computer 104 via use of the portable media device 132.

Next, in process step 410, the healthcare provider computer 104 is configured to calculate the position of the relevant bones or bony anatomy of the patient based on the sensor data. For example, in the illustrative embodiment of FIGS. 1 and 2, the healthcare provider computer 104 is configured to determine the position of the femur and the tibia of the patient relative to each other based on the sensor data generated by the position sensors 92 of the sensor circuits 30, 32, 34, 36. In embodiments in which the position sensors 92 are embodied as accelerometers generating data indicative of the position of the position sensor 92 relative to the Earth's gravitational field, the healthcare provider computer 104 may determine the relative position of the patient's femur and tibia by comparing the sensor data generated by those sensor circuits 30, 34 positioned on the superior half of the knee sleeve 14 and those sensor circuits 32, 36 positioned on the inferior half of the knee sleeve 14.

Once the position of the relevant bones or bony anatomy of the patient is determined in step 410, rendered images of the relevant bones of the patient are displayed to the healthcare provider (e.g., orthopaedic surgeon or physical therapist) via the display device 126. To do so, in some embodiments, the healthcare provider computer 104 is configured to generate an image of each relevant bone (e.g., of the femur and tibia of the patient) and display such images in a position relative to each other as determined in process step 410. Additionally or alternatively, the healthcare provider computer 104 may be configured to retrieve pre-operative or otherwise pre-generated medical images of the relative bones of the patient from an image database and display images of the patient's bones based on such pre-operative or pre-generated medical images. In this way, the rendered images may closely match the actual physical structure of the bones of the patient.

It should be appreciated that such displayed images of the bones or boney anatomy are updated as new sensor data is received from the patient computer 104 (or from the sensing circuit 16 of the knee sleeve 14 in some embodiments). In this way, the motion of the patient's joint (e.g., knee joint 12) may be remotely or locally monitored by the healthcare provider. Additionally, the healthcare provider computer 104 may determine the range of motion of the patient's joint based on the position sensor data by, for example, determining the maximum flexion and extension angles between the respective bones. The range of motion may also be actively monitored by the healthcare provider using the updated images of the relevant bones displayed on the display device 126. For example, the healthcare provider may determine whether the patient is fully extending and/or flexing the relevant joint based on such images.

In some embodiments, the healthcare provider computer 104 is configured to determine additional data in process step 414. For example, the healthcare provider computer 104 may be configured to determine the temperature of the patient based on sensor data generated by one or more temperature sensors 94. Additionally or alternatively, the healthcare provider computer 104 may be configured to determine the heart rate of the patient based on sensor data generated by one of more heart rate sensors 96. Further, in some embodiments, the healthcare provider computer 104 may be configured to determine a cycle count indicative of the number of times the patient's joint (e.g., knee joint 12) has been cycled between two or more predetermined positions (e.g., between a flexion position and a extension position). The healthcare provider computer 104 may determine the cycle count based on, for example, the range of motion and/or images generated in process steps 410, 412. Subsequently, in process step 416, the healthcare provider computer 104 is configured to display the additional data (e.g., the temperature data, the heart rate data, the cycle count, etc.) determined in process step 414. To do so, the healthcare provider computer 104 may display the additional data to the healthcare provider via the display device 126. In this way, the healthcare provider may remotely or locally monitor the range of motion, the cycle count, the temperature, and/or the heart rate of the patient.

It should be appreciated that the system 100 allows the healthcare provider to remotely and/or locally monitor the kinematic motion of the patient. As such, the healthcare provider may monitor or analyze any aspect of the kinematic motion or other data determinable based on the kinematic motion of the patient such as, for example, the range of motion achieved by the patient, the cycle count achieved by the patient, the rehabilitative progress of the patient, aspects of the patient's gait, and the like. In addition, such monitoring or analysis may be performed by the healthcare provider pre-operatively and/or post-operatively in those embodiments in which an orthopaedic surgical procedure is performed. Similarly, such monitoring or analysis may be performed by the healthcare provider pre-therapy and/or post-therapy in those embodiments in which the patient is assigned a physical therapy regime. In addition, the healthcare provider may monitor the progress of the patient's physical therapy as the patient is performing the assigned exercises. As such, by remotely and/or locally monitoring the patient's performance, the healthcare provider is able, for example, to verify that the patient is adhering to the physical therapy regimen prescribed by the healthcare provider.

In some embodiments, the healthcare provider may be in communicative contact with the patient (for example, via a telephone) while the patient is performing the assigned exercises. In such embodiments, the healthcare provider may provide additional instructions to the patient to ensure that the patient is performing the exercises correctly. For example, the healthcare provider may monitor the range of motion of the patient's joint in real time or near real time via the display device 126. If the healthcare provider determines that the patient is not achieving a satisfactory range of motion, the healthcare provider can verbally instruct the patient to extend or flex the joint further and subsequently monitor the improved range of motion of the joint as indicated via the healthcare provider computer 104. In this way, the healthcare provider may provide an amount of healthcare to the patient without the requirement that the patient visit the healthcare provider's office or that the healthcare provider visit the residence of the patient. As such, in some embodiments, the healthcare provider computer 104 may be configured to record the monitoring session, generate charts or graphs of the patient's improvement over time, and generate a bill for the time spent by the healthcare provider. The generated bill, along with records of the monitoring sessions and charts illustrating improvement of the patient, may be subsequently provided to a third party, such as an insurance provider, for payment thereof.

Although the apparatus 10 has been described herein in reference to a knee sleeve, it should be appreciated that other garments may be used in other embodiments. For example, in some embodiments the garment may be embodied as a foot-covering garment, an elbow-covering garment, a wrist-covering garment, a shoulder- and/or back-covering garment, or a hip-covering garment. That is, any garment may be used that does not significantly alter the natural movement of the patient, is form fitting to the relative portion of the patient's anatomy such that the garment sufficiently tracks the movement of the relevant bones, and to which the sensing circuitry 16 may be coupled such that at least one sensor circuit 30, 32, 34, 36 is positioned on either side of the relevant joint. For example, in embodiments in which the garment is embodied as an ankle-covering garment, the sensor circuits 30, 34 may be coupled to a superior half of the ankle-covering garment while sensor circuits 32, 36 are coupled to an inferior half of the ankle-covering garment. Similarly, in embodiments in which the garment is embodied as an elbow-covering garment, the sensor circuits 30, 34 may be coupled to a superior half of the elbow-covering garment while sensor circuits 32, 36 are coupled to an inferior half of the elbow-covering garment. In embodiments in which the garment is embodied as a wrist-covering garment, the sensor circuits 30, 34 may be coupled to a superior half of the wrist-covering garment while sensor circuits 32, 36 are coupled to an inferior half of the wrist-covering garment as defined by a plane bisecting the wrist joint of the patient when the glove is worn. Additionally, in embodiments in which the garment is embodied as a shoulder- and/or back-covering garment, the sensor circuits 30, 34 may be coupled to a superior half of the garment while sensor circuits 32, 36 are coupled to an inferior half of the garment as defined by a plane bisecting any one of the joints of the patient's spine and/or shoulder joint. Further, in embodiments in which the garment is embodied as a hip-covering garment, the sensor circuits 30, 34 may be coupled to a superior half of the hip-covering garment while sensor circuits 32, 36 are coupled to an inferior half of the hip-covering garment as defined by a plane bisecting the hip joint of the patient when the shorts are worn by the patient.

## Claims

1. Apparatus for monitoring the range of motion of a patient's knee, the apparatus comprising:
a sleeve configured to be worn around a patient's limb, the sleeve having a superior half and an inferior half defined by a bisecting plane;
a first sensor circuit coupled to the superior half of the sleeve, the first sensor circuit including a first sensor configured to generate first data indicative of the position of the first sensor;
a second sensor circuit coupled to the inferior half of the sleeve, the second sensor circuit including a second sensor configured to generate second data indicative of the position of the second sensor; and
a communication circuit coupled to the sleeve and electrically coupled to the first sensor circuit and the second sensor circuit, the communication circuit being configured to transmit the first and second data.

2. The apparatus of claim 1, in which the first sensor circuit, the second sensor circuit, and the communication circuit are removably coupled to the sleeve.

3. The apparatus of claim 1, in which the sleeve includes an aperture and at least one of the first sensor circuit and the second sensor circuit is sized to be positioned in the aperture such that a portion of the at least one of the first sensor circuit and the second sensor circuit is in contact with the skin of the patient when the sleeve is worn by the patient.

4. The apparatus of claim 3, in which the at least one of the first sensor circuit and the second sensor circuit comprises a third sensor configured to generate third data indicative of the temperature of the patient.

5. The apparatus of claim 1, in which:
(i) the superior half of the sleeve includes a first aperture and the inferior half of the sleeve includes a second aperture,
(ii) the first sensor circuit is sized to be positioned in the first aperture such that a portion of the first sensor circuit is in contact with the skin of the patient when the sleeve is worn by the patient, and
(iii) the second sensor circuit is sized to be positioned in the second aperture such that a portion of the second sensor circuit is in contact with the skin of the patient when the sleeve is worn by the patient.

6. The apparatus of claim 5, in which at least one of the first sensor circuit and the second sensor circuit comprises a third sensor configured to generate third data indicative of the temperature of the patient.

7. The apparatus of claim 1, in which:
(i) the first sensor circuit includes a third sensor configured to generate third data indicative of the localized skin temperature of the patient,
(ii) the second sensor circuit includes a fourth sensor configured to generate fourth data indicative of the localized skin temperature of the patient, and
(iii) the communication circuit is configured to transmit the third and fourth data.

8. The apparatus of claim 7, in which:
(i) the first sensor circuit includes a fifth sensor configured to generate fifth data indicative of the heart rate of the patient,
(ii) the second sensor circuit includes a sixth sensor configured to generate sixth data indicative of the heart rate of the patient, and
(iii) the communication circuit is configured to transmit the fifth and sixth data.

9. The apparatus of claim 1, in which the communication circuit comprises a memory device, the communication circuit being configured to store the first and second data in the memory device.

10. The apparatus of claim 1, in which the communication circuit comprises a memory device, the communication circuit being configured to retrieve stored data indicative of the position of at least one of the first and the second sensor from the memory device and transmit the stored data.

11. The apparatus of claim 1, which includes:
(i) a third sensor circuit coupled to the superior half of the knee sleeve, the third sensor circuit including a third sensor configured to generate third data indicative of the position of the third sensor; and
(ii) a fourth sensor circuit coupled to the inferior half of the knee sleeve, the fourth sensor circuit including a fourth sensor configured to generate fourth data indicative of the position of the fourth sensor.

12. The apparatus of claim 11, in which:
(i) the first sensor circuit and the second sensor circuit are coupled to a lateral side of the knee sleeve, and
(ii) the third sensor circuit and the fourth sensor circuit are coupled to an anterior side of the knee sleeve.

13. The apparatus of claim 1, in which the communication circuit comprises a first transmitter electrically coupled to the first sensor and a second transmitter electrically coupled to the second sensor.

14. The apparatus of claim 1, in which the sleeve is a knee sleeve which can be worn around a patient's knee.

15. A system for monitoring the range of motion of a patient's joint, the system comprising:
a sleeve;
a first sensor coupled to the sleeve and configured to generate first data indicative of the position of the first sensor;
a second sensor coupled to the sleeve and configured to generate second data indicative of the position of the second sensor;
a communication circuit coupled to the sleeve and electrically coupled to the first sensor and the second sensor, the communication circuit being configured to transmit the first and second data wirelessly; and
a first computer configured to determine third data indicative of the position of a first bone which articulates at the said joint based on the first data and fourth indicative of the position of a second bone which articulates at the joint with the first bone based on the second data.

16. The system of claim 15, in which the first computer is configured to display indicia of the first bone on a display device in a position based on the third data and indicia of the second of the patient on the display device in a position based on the fourth data.

17. The system of claim 15, which includes a second computer configured to receive the first data and the second data from the communication circuit and transmit the first data and the second data to the first computer over a network.

18. The system of claim 15, which includes a second computer configured to display indicia of the first bone on a display device in a position based on the third data and indicia of the second bone on the display device in a position based on the fourth data, in which the first computer is configured to transmit the third data and the fourth data to the second computer over a network.

19. The system of claim 15, which includes a second computer including a portable media device, the second computer being configured to receive the first data and the second data from the communication circuit and store the first data and the second data on the portable media device.

20. The system of claim 19, in which the first computer is configured to retrieve the first and second data from the portable media device and display indicia of the femur of the patient on a display device in a position based on the third data and indicia of the tibia of the patient on the display device in a position based on the fourth data.

21. The system of claim 15, in which the first computer is configured to determine a cyclic count indicative of the number of times the patient's joint is moved between a flexion angle and an extension angle within a period of time based on the first data and the second data.

22. The system of claim 15, which includes :
a third sensor coupled to the sleeve and configured to generate fifth data indicative of the position of the third sensor;
a fourth sensor coupled to the sleeve and configured to generate sixth data indicative of the position of the fourth sensor,
in which the first and third sensors are coupled to a lateral side of the sleeve, the second and fourth sensors are coupled to an anterior side of the sleeve, and the communication circuit is configured to wirelessly transmit the first, second, fifth, and sixth data.

23. The system of claim 15, in which the communication circuit comprises a first transmitter electrically coupled to the first sensor and a second electrically coupled to the second sensor.
